# EUROPEAN PATENT APPLICATION

(11) **EP 2 848 278 A1**
(43) Date of publication of application: **18.03.2015**
(21) Application number: 14181078.8
(22) Date of filing: 14.08.2014
(51) Int. Cl.: A61M 25/00, A61M 39/10

(54) **Drainage catheter and assembly with rotational fitting and methods of use**

(30) Priority: 15.08.2013 US 201313967774
(71) Applicant: Cook Medical Technologies LLC, Bloomington, IN 47404 (US)
(72) Inventor: Miller, Christopher, Danville, Indiana 46122 (US); Stewart, Kyle, Cincinnati, Ohio 45219 (US)
(74) Representative: Garratt, Peter Douglas

(57) **Abstract**

A catheter (8) having a longitudinal body (10) includes a rotational fitting (34) located thereon, thus defining a distal longitudinal body portion (38) distal of the fitting and a proximal longitudinal body portion (42) proximal of the fitting. The respective proximal and distal longitudinal body portions are independently moveable from each other such that movement of one of the proximal and distal longitudinal body portions of the catheter, such as during removal and/or replacement of a urine drainage bag from the proximal end of the catheter, does not impart movement to the distal longitudinal body portions of the catheter that may be located in the patient's urethra and bladder. Methods of handling a drainage catheter and for removing a fluid collection bag (30) from a catheter assembly (2) are also disclosed.

## Description

### Technical Field

The present invention relates generally to catheters and methods of using the same, and more specifically, to drainage catheters intended for drainage of urine or other bodily fluids.

Drainage catheters are widely used when patients are unable to reliably void themselves, typically after surgery that is related to a urinary function. For patients of either sex, a urinary catheter may be needed during or after surgery and/or when the patient is immobilized. These catheters often take the form of what is known as a Foley catheter, which includes a drainage lumen that extends from a distal end that is held in the bladder to a proximal end that remains outside the body. Fluid draining from the patient may be collected in a container or bag that is in communication with the proximal end of the catheter.

Once a catheter has been placed within the urethra and into the bladder, it may remain there, indwelling in the patient, sometimes as long as several days, after which time it is removed to avoid bacterial growth and infection in the urinary tract. However, during the time that the catheter is indwelling, the patient has a constant flow of urine from the bladder, through the catheter, and into a urine drainage bag that is attached to the proximal end of the catheter and carried by the patient. The drainage bag must therefore be removed and replaced fairly frequently, as needed or desired, such as when the drainage bag becomes full.

Even slight movement of the catheter while it is indwelling may cause discomfort in the patient's urethra and/or bladder. Torsional movement that is caused, for example, by adjustments of the catheter or by detaching the urine drainage bag from the catheter may frequently cause discomfort to the patient, as the urine bag must be changed up to several times per day.

Accordingly, it is desirable to provide a catheter including a rotating mechanism or fitting for reducing or substantially eliminating torsional and/or angular movement of the portion of the catheter located in the patient's urethra and bladder.

### Summary

The present disclosure describes a catheter and a catheter assembly. In one example, the catheter comprises a longitudinal body having a proximal end and a distal end and at least one drainage lumen extending therebetween. A rotatable fitting or a universal joint fitting is located on the longitudinal body of the catheter, thus defining a distal longitudinal body portion distal of the fitting and a proximal longitudinal body portion proximal of the fitting, such that the proximal and distal longitudinal body portions are independently moveable with respect to each other.

A catheter assembly is also described. In one example, the assembly comprises a catheter comprising a longitudinal body having a proximal end and a distal end and at least one drainage lumen extending therebetween. A fitting is located on the longitudinal body of the catheter, thus defining a distal longitudinal body portion distal of the fitting and a proximal longitudinal body portion proximal of the fitting, wherein the proximal and distal longitudinal body portions are independently moveable with respect to each other. A fluid collection bag is removably attachable to the proximal end of the catheter.

A method for handling a drainage catheter is also described. The method comprises maneuvering a fluid collection bag removably attached to a drainage the catheter, the drainage catheter comprising a longitudinal body having a proximal end and a distal end and at least one drainage lumen extending therebetween; a fitting located on the longitudinal body of the catheter, thus defining a distal longitudinal body portion distal of the fitting and a proximal longitudinal body portion proximal of the fitting, wherein the proximal and distal longitudinal body portions are independently moveable with respect to each other such that movement of the proximal longitudinal body portion during the maneuvering of the fluid collection bag does not impart movement or torsion to the distal longitudinal body portion of the catheter.

A method for detaching a fluid collection bag of a drainage catheter assembly is also described. The method comprises providing a catheter assembly comprising a drainage catheter comprising a longitudinal body having a proximal end and a distal end and at least one drainage lumen extending therebetween; a fitting located on the longitudinal body of the catheter, thus defining a distal longitudinal body portion distal of the fitting and a proximal longitudinal body portion proximal of the fitting, wherein the proximal and distal longitudinal body portions are independently moveable with respect to each other; and a fluid collection bag removably attached to the proximal end of the catheter. The method further comprises manipulating the fluid collection bag to detach it from the catheter, wherein movement of the proximal longitudinal body portion during manipulation of the fluid collection bag does not impart movement or torsion to the distal longitudinal body portion of the catheter.

### Brief Description of the Drawings

Figure 1 is a front perspective view of one example of a catheter assembly in use with a portion of a catheter indwelling in a patient's bladder and urethra.
Figure 2 is a front elevation of one example of a catheter according to the present disclosure having a proximal end in communication with a urine collection bag.
Figure 3 is a front elevation of another example of a catheter according to the present disclosure in communication with a urine collection bag via an intermediate tubing segment extending between a proximal end of the catheter and the collection bag.
Figure 4 is an enlarged perspective view of a rotational fitting or mechanism located on a portion of the longitudinal body of a catheter.
Figure 5 is an exploded view of a rotational fitting or mechanism that may be attached to a portion of the longitudinal body of a catheter.
Figure 6 is a front perspective of one example of a catheter assembly in use with a distal portion of a catheter indwelling in a patient's bladder and urethra and a urine collection bag detachable from a proximal end of the catheter.

### Detailed Description

Throughout this specification, the terms proximal and proximally are used to refer to a position or direction away from, or even external to a patient's body and the terms distal and distally are used to refer to a position or direction towards the patient and/or to be inserted into a patient's body orifices or cavities.

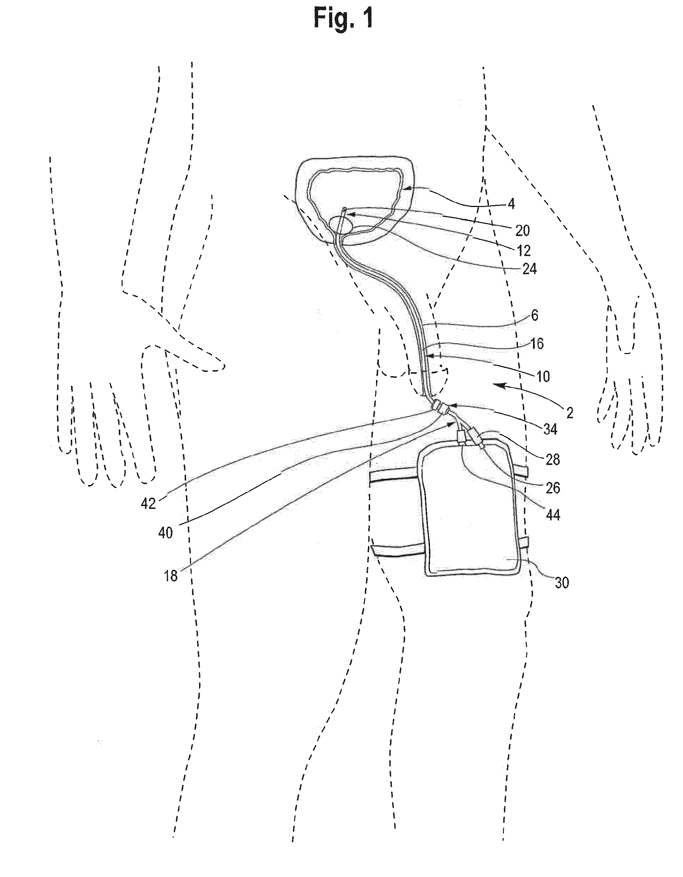

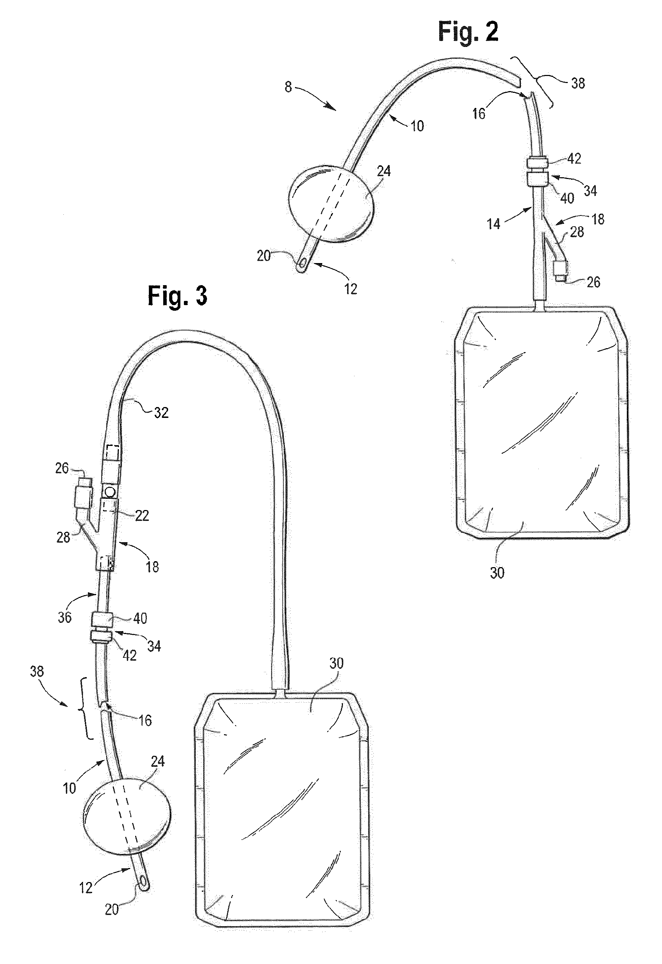

Figure 1 illustrates one example of a catheter assembly 2 intended for placement in the bladder 4 and urethra 6 of a patient for drainage of urine. Its dimensions are alterable so that it may be appropriately dimensioned to navigate to the bladder, or any other target body cavity, from which fluid will be drained. As shown in Figures 2 and 3, the assembly 2 preferably includes a catheter 8 having a longitudinal body 10 having a distal end 12 and a proximal end 14. There is a drainage lumen 16 extending along the length of the longitudinal body 10 between the proximal 14 and distal 12 ends and, in one example, a connector 18, which may be in the shape of a "Y" located at the proximal end 14 of the catheter 8. The connector 18 may be integrally formed with and/or part of the longitudinal body as shown in Figure 2 or, alternatively, the connector 18 may be a separately formed or molded component attached to the proximal end 14 of the catheter as shown in Figure 3. The catheter 8 may include one or more openings 20 at or near its distal end 12, such that when the distal end 12 of the catheter 8 is positioned in the bladder 4, the openings 20 allow urine to enter and flow through the drainage lumen 16 and also through a drainage bore or lumen 22 extending through the connector 18. The catheter 8 may also include additional ports or orifices at various points along the longitudinal body to allow urine to enter the catheter 8. Radiopaque markers (not shown) may optionally be located on longitudinal body 10 to aid in the positioning of catheter 8 using a fluoroscope or similar device.

The catheter 8 may include a mechanism to help retain the catheter in place once it has been properly positioned in the urethra 6 and bladder 4. In one example, the retention mechanism includes a balloon 24. Once the catheter has been placed within the bladder 4 of the patient, the balloon 24 may be inflated to hold or otherwise anchor the catheter 8 in place. The balloon 24 may be inflated with fluid such as saline or other medically acceptable fluid through an inflation port 26 and inflation lumen 28. Inflation lumen 28 extends from port 26 through the connector 18 and along the length of the catheter body 10 into balloon 24. The inflation lumen 28 may run parallel with the drainage lumen 16, but preferably, the two lumens 16, 28 remain separate for their entire lengths.

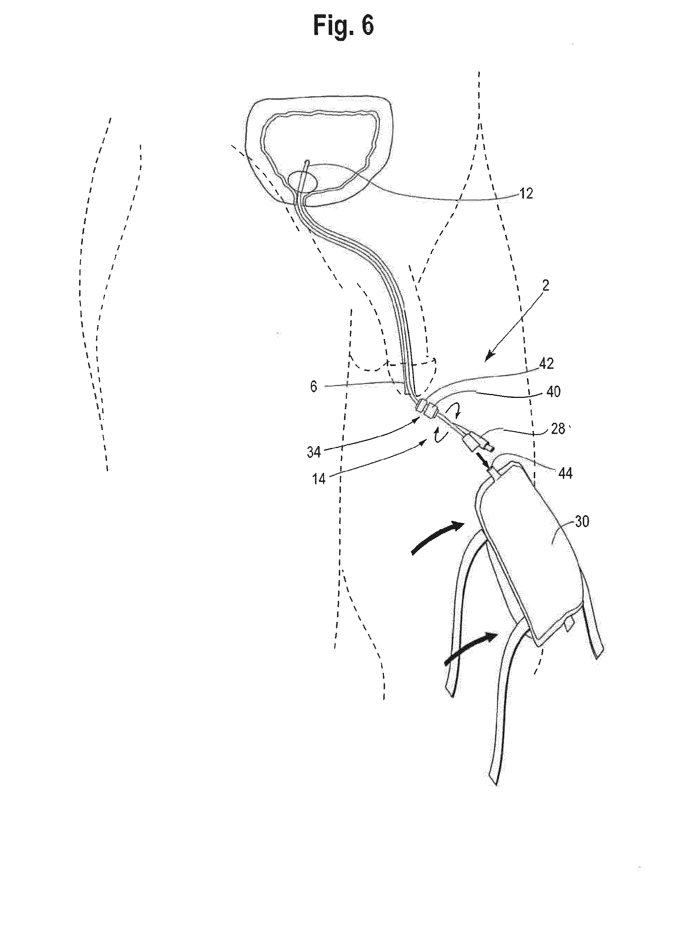

The catheter assembly 2 may further include a downstream drainage or collection bag 30, such as a urine collection bag located at the proximal end 14 of the catheter 8. The collection bag 30 may be directly attached to the connector 18 at the proximal end 14 of the catheter 8 as shown in Figure 2, or, alternatively, the collection bag 30 may be attached to the proximal end 14 of the catheter 8 via an intermediate tubing segment 32, as shown in Figure 3. As Figure 1 shows, urine draining from the bladder 4 flows through the catheter drainage lumen 16 , through the drainage lumen 22 in the connector 18 and into the drainage bag 30 (or, as shown in Figure 3, through the intermediate tubing segment 32 and then into the bag 30). Oftentimes, the urine collection bag 30 is strapped to a patient's leg as shown in Figure 6. This allows the bag 30 to be securely held close to and supported by the patient's body.

As shown best in Figures 2 and 3, the catheter 8 preferably includes a fitting or structure 34 located on the longitudinal body 10. In one example, the structure is a fitting 34 that facilitates rotation of one portion of the longitudinal body relative to another portion of the longitudinal body. More specifically, the fitting 34 may separate the longitudinal body 10 of the catheter 8 into a proximal body portion 36 and a distal body portion 38. Preferably, the fitting 34 is located on the longitudinal body 10 closer to the proximal end 14 of the catheter such that when the distal end 12 of the catheter is inserted into the urethra 6 and located within the bladder 4, the fitting 34 remains outside of the patient's body. Preferably, the rotatable fitting 34 is positioned on the longitudinal body 10 of the catheter 8 distal to, or upstream, of the connector 18. Alternatively, the rotatable structure 34 may, in another example, be located on the connector 18, on the intermediate tubing segment 32 leading to the urine collection bag 30, or, in yet a further example, more than one rotatable structure 34 may be placed on one or more of the catheter body 10, the connector 18 and/or the intermediate tubing segment 32, or a combination thereof, to facilitate relative rotation among and between these various components of the catheter assembly 2.

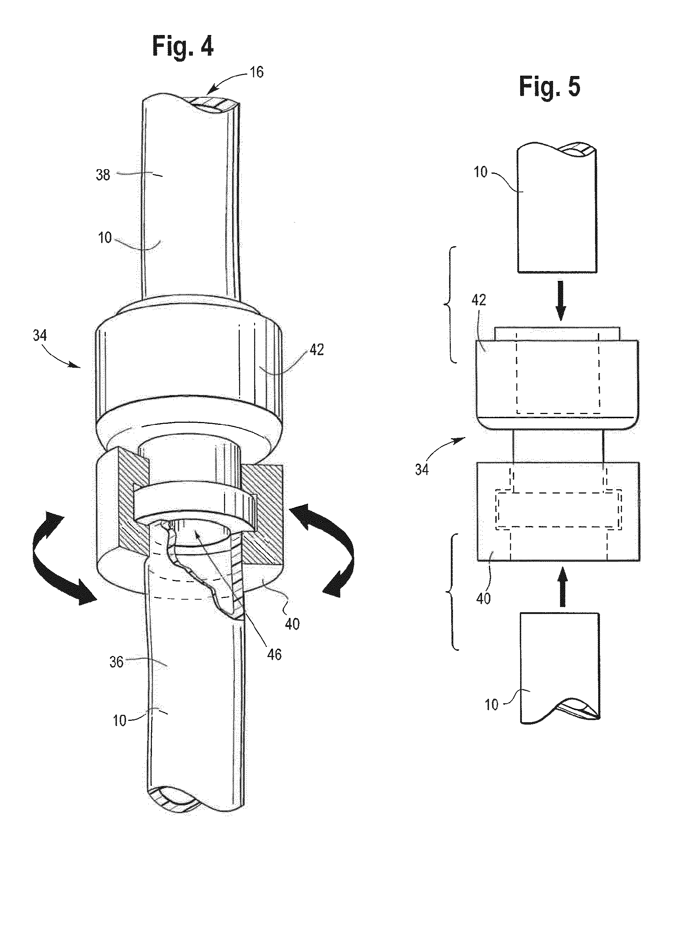

In one example, as illustrated in Figure 4, the fitting 34 may include two segments that are configured to securely connect to each other. A first segment 40 may be attached to the proximal catheter body portion 36 and the second segment 42 attached to the distal catheter body portion 38. One segment may be a female fitting while the other segment is a correspondingly shaped male fitting so that the male and female segments can be joined together. It is also contemplated that the fitting 34 may be constructed from other variously shaped and sized and releasably connectable segments or components that can rotate with respect to each other, such as, in one non-limiting example, a ball bearing, a ball and socket and other hinge-like or universal joint structures. In yet another example, the fitting 34 may be constructed from a single, unitary piece of resilient material (as opposed to two separately joined segments) that may be hinged, flexible, bendable and/or rotatable. Whether the fitting is one unitary component or comprises multiple segments, there is preferably a lumen 46 extending through the fitting to provide flow communication through the catheter drainage lumen 16 between the proximal and distal catheter body portions. The fitting 34 may be constructed from a variety of materials such as stainless steel, plastic, rubber, a combination thereof, or any other biocompatable material which allows relative rotation of the respective proximal 36 and distal 38 catheter body segments. Alternatively, if the fitting 34 were to be placed on the connector 18 and/or on the intermediate tubing segment 32, the fitting 34 preferably allows relative rotation between the portion of the distal longitudinal catheter body 38 that is located in the urethra/bladder and the portion of the catheter assembly 2 that is proximal to, or outside of the patient's body, so that movement and/or rotation of the external assembly components does not impart movement to the indwelling portion of the catheter 8.

Preferably, the respective first and second fitting segments 40, 42 have full 360° rotation with respect to each other. It is desirable that the fitting 34 facilitates axial rotation between the proximal and distal catheter body segments 36, 38 so that they may twist or spin, along a longitudinal axis, relative to each other. It is also desirable that the fitting 34 facilitates angular rotation of the proximal and distal catheter body segments 36, 38 in addition to the axial rotation, thereby allowing a range of movement between the respective catheter segments 36, 38. In one example, the fitting 34 allows the proximal catheter body segment 36 to be moved or rotated separate and independent from the distal catheter body segment 38. In other words, movement or rotation of the proximal catheter body segment 36 will not impart movement or rotation of the distal catheter body segment 38, which if such movement were to translate up along the length of the catheter 8 located in the urethra 6 and bladder 4, may cause pain or discomfort to the patient.

In one non-limiting example, the urine collection bag 30 must be removed from the catheter assembly 2 and replaced as necessary or desired. Replacement of the collection bag 30 may occur frequently, such as several times a day and/or when the collection bag 30 becomes full. To replace the bag 30, the bag (or, if present, the intermediate tubing segment 32 connecting the collection bag 30 to the connector 18) may be detached from the connector 18 and the used/full bag 30 discarded. A replacement (empty) bag 30 (with or without an intermediate tubing segment 32 attached to the distal end 44 of the bag), can be re-attached to the connector 18. Bag detachment and reattachment may require twisting and pulling at the attachment site that is external to the patient in order to separate the bag 30 (and/or tubing segment 32) from the portion of the catheter assembly 2 that remains connected to or is indwelling within the patient.

It will be appreciated that the rotatable fitting 34 as described herein may allow the urine collection bag 30 (and, when present, the intermediate tubing segment 32 connecting the collection bag 30 to the connector 18) to move independently from at least a portion of the catheter body 10. For example, the fitting 34 may permit the collection bag 30 and tubing segment 32 to move with the patient, as may occur with normal physical activity. The fitting 34 may also allow the patient to maneuver the proximal end of the catheter body 36, the tubing segment 32 and collection bag 30, all while reducing or substantially eliminating torque and other movement of the catheter within the patient's urethra and bladder.

The rotatable fitting 34 may be incorporated into the catheter assembly 2 during manufacture, or alternatively, may be added later by a physician or patient. For example, starting with a standard catheter, such as a Foley catheter, the longitudinal body 10 can be cut, split or otherwise severed at a desired location where the fitting 34 will be placed as illustrated in Figure 5. Preferably, the longitudinal body 10 can be cut near the proximal end 14, at a point that will remain external to the patient. The loose ends of the longitudinal body 10 can then each be secured to each side or segment 40, 42 of the fitting 34, as shown for example, in Figure 5. The fitting 34 can be secured to the respective proximal and distal portions 36, 38 of the longitudinal catheter body 10 by various known mechanisms, including, but not limited to adhesives, bonding, screws, threads and other attachment means, or, alternatively, the fitting 34 can be integrally molded to the respective ends of the proximal and distal longitudinal catheter body segments 36, 38.

In addition to use with a catheter 8, embodiments of the present invention are equally useful in providing relative rotation between respective portions of any medical device where one portion of a device has been introduced into a patient and/or a body cavity (either temporarily or that remains indwelling) while a second portion remains external to a patient, thus facilitating movement and maneuverability of the external portion while reducing or eliminating movement of the internal or indwelling portion. Elimination of movement and torque of the internal portion of a medical device while allowing the external portion of the device to be manipulated, by providing a rotatable fitting as described herein, provides various advantages, including, but not limited to reducing and eliminating pain or discomfort for the patient.

Throughout this specification, unless the context requires otherwise, the words "comprise" and "include" and variations such as "comprising" and "including" will be understood to imply the inclusion of an item or group of items, but not the exclusion of any other item or group items.

While various embodiments of the invention have been described, it will be apparent to those of ordinary skill in the art that many more embodiments and implementations are possible within the scope of the invention. Furthermore, although various indications have been given as to the scope of this invention, the invention is not limited to any one of these but may reside in two or more of these combined together. Accordingly, the invention is not to be restricted except in light of the attached claims and their equivalents.

## Claims

1. A catheter comprising:
a. a longitudinal body having a proximal end and a distal end and at least one drainage lumen extending therebetween;
b. a fitting located on the longitudinal body of the catheter, thus defining a distal longitudinal body portion distal of the fitting and a proximal longitudinal body portion proximal of the fitting,
c. wherein the proximal and distal longitudinal body portions are independently moveable with respect to each other.

2. The catheter of claim 1 wherein the fitting comprises a rotatable fitting.

3. The catheter of any one of the preceding claims wherein the at least one drainage lumen extends though the fitting.

4. The catheter of any one of the preceding claims wherein at least a portion of the catheter distal end is configured for insertion into a patient's urethra and bladder.

5. The catheter of any one of the preceding claims wherein the fitting is located on the proximal longitudinal body portion and remains external to a patient's body when at least a portion of the catheter distal end is inserted into a patient's urethra and bladder.

6. The catheter of claim 1 wherein the fitting comprises a unitary piece of resilient material.

7. The catheter of any one of the preceding claims wherein the fitting comprises a first segment and a second segment.

8. The catheter of claim 7 wherein one of the first and second fitting segments is attached to the distal longitudinal body portion of the catheter and the other of the first and second fitting segments is attached to the proximal longitudinal body portion of the catheter.

9. The catheter of claim 7 or claim 8 wherein one of the first and second fitting segments is a male fitting and the other of the first and second fitting segments is a correspondingly shaped female fitting.

10. The catheter of any one of claim 7 to 9 wherein the first and second fitting segments are rotationally and angularly moveable with respect to each other.

11. The catheter of any one of the preceding claims wherein movement of one of the proximal and distal longitudinal body portions of the catheter does not impart movement to the other of the proximal and distal longitudinal body portions of the catheter.

12. The catheter of any one of the preceding claims wherein the fitting is configured to facilitate substantially independent movement of the respective proximal and distal longitudinal catheter body portions.

13. A catheter assembly comprising a catheter of any one of the preceding claims and a fluid collection bag removably attachable to the proximal end of the catheter.

14. The assembly of claim 13 further comprising a branched connector on the proximal end of the catheter, the connector defining a drainage lumen; wherein the connector drainage lumen is in communication with the catheter drainage lumen.

15. The catheter assembly of claim 13 or claim 14 further comprising an intermediate tubing segment providing communication between the fluid collection bag and the proximal end of the catheter.
